# EUROPEAN PATENT APPLICATION

(11) **EP 1 947 197 A1**
(43) Date of publication of application: **23.07.2008**
(21) Application number: 07016658.2
(22) Date of filing: 24.08.2007
(51) Int. Cl.: C12Q 1/68

(54) **Method for the amplification of at least one nucleic acid**

(71) Applicant: Siemens Aktiengesellschaft, 80333 München (DE)
(72) Inventor: Schreiter, Matthias, 81379 München (DE); Stanzel, Manfred, Dr., 91056 Erlangen (DE)

(57) **Abstract**

A method for the amplification of at least one nucleic acid comprises the following method steps:
a) provision of a gravimetric sensor, first primers of a primer pair that are at least partly complementary to the nucleic acid being immobilized on the surface of the sensor,
b) hybridization of the nucleic acid with the first primer,
c) extension of the first primer and
d) measurement of the mass increase during or after the extending of the first primer.

The proposed method can be used for real-time PCR without using labeled primers.

## Description

The present invention relates to a method for the amplification of at least one nucleic acid in a sample.

The detection of extremely small quantities of nucleic acids (e.g. DNA, cDNA or RNA) has continually gained in importance in recent years. This detection has become possible through the use of the so-called polymerase chain reaction (PCR) since the quantity of nucleic acid present is doubled in this method under optimum conditions during each reaction cycle.

The PCR is based on the replication of nucleic acids with the aid of thermostable DNA polymerases. In this case, a pair of oligonucleotide primers (single-stranded oligonucleotides) is brought into contact with the nucleic acid to be amplified. The primers are chosen such that they bind at the two ends of a fragment to be amplified on the complementary strands. During the elongation, the primers are then elongated along the respective target nucleic acid strand in the 3' direction (forward and backward primers). Forward and backward primers are alternatively also referred to as sense or antisense primers. In this way, the portion lying between the sites complementary to the primers on the target nucleic acid can be amplified. For subsequent detection reactions, the PCR products are advantageously separated from primers, nucleotides and other disturbing components of the PCR mixture.

The PCR sequence comprises a plurality of thermocycles each having three steps. The first step involves heating (e.g. to 94°C) in order to separate the strands of the double stranded target DNA contained in the sample (denaturation). The temperature is thereupon lowered (e.g. to 45 - 60°C), such that the primers can attach to the complementary sequences of the DNA single strands (annealing). In the last step, the DNA section between the primers is filled by the DNA polymerase with the respectively complementary nucleotides (elongation, e.g. at 72°C). This cycle is run through typically approximately 15 - 50 times during a PCR. The temperatures specified serve merely as exemplary values and should be coordinated with the PCR that is to be respectively specifically performed.

Consequently, from a small number of specimens of a DNA (or other nucleic acid) present, a multiplicity of copies can be produced within a very short time, the concentration of said copies sufficing for the subsequent qualitative detection of the DNA. By way of example, after running through the PCR 20 times (typical time requirement 20 to 40 minutes), 2²⁰ times, that is to say approximately 10⁶ times, the quantity of the nucleic acid originally used is obtained. However, the amplification rate, averaged over the entire PCR, per cycle is only theoretically of the value 2, which would correspond to a doubling per cycle. In general, the efficiency is significantly lower (approximately 1.75 to 1.9). Since the actual efficiency is unknown it is not possible to calculate back from the final concentration of amplified DNA obtained after the PCR to the quantity of target nucleic acid previously present. However, a series of applications are known in which it is necessary to determine the concentration of nucleic acids. Examples thereof are determination of the microbe count, control of HIV therapy and gene expression analysis. There are numerous methods for quantitatively determining the number of copies of the nucleic acid(s) (to be amplified) used, which methods are generally known by the designation "real-time PCR".

These real-time PCRs are replication methods for nucleic acids which are based on the principle of the conventional PCR and additionally afford the possibility of quantification. In this case, the quantification is often based on fluorescence measurements carried out after or during the respective PCR cycles, the fluorescence increasing proportionally with the quantity of PCR products generated. By way of example, so called intercalation dyes (e.g. Sybr Gr) can be used for this as fluorescent dyes. These specific fluorescent dyes intercalate into the nucleic acid double strand or bind to double-stranded DNA, whereby the fluorescence of said dyes increases. The increase in the nucleic acid to be replicated is therefore correlated with an increase in fluorescence from cycle to cycle of the PCR. What is disadvantageous about this method is that no multiplexability is provided here, that is to say it is not possible to distinguish between different species of PCR products.

As an alternative, solutions are known which use so-called fluorescence resonance energy transfer (FRET) in order to enable a quantification of the nucleic acids present. In the fundamental physical process, energy from an excited fluorescent dye (donor fluorophor) is transferred to a second fluorescent dye (acceptor fluorophor) in radiationless fashion. The intensity of the FRET is dependent, inter alia, on the distance between these two fluorophors. If the distance between the two fluorophors increases, then the fluorescence signal of the acceptor decreases, while the fluorescence signal of the donor increases. Numerous methods are known which utilize FRET for the quantification of nucleic acids. The so-called hybridization probes (e.g. LightCycler^{®}), hydrolysis probes (e.g. TaqMan^{®}) molecular beacons, and scorpion primers shall be mentioned as examples. All these methods use marked nucleic acid probes which change their fluorescence depending on binding or non-binding to the nucleic acid. In general, said probes are constructed in such a way that they hybridize within the DNA section to be copied. During the extending reaction of the PCR, the probes are at least partly destroyed by the activity of the DNA polymerase and removed from the template, whereby the fluorescence activity is altered. The release of said probes can thus be read by means of fluorescence-optical methods, which in turn permits conclusions about the quantity of PCR product formed. By means of the parallel amplification of corresponding standards of predetermined concentration it is possible, finally, to determine the initial concentration of the nucleic acid.

What is common to all the known methods of real-time PCR is that a so-called "label", that is to say a marking, is used for the detection and quantification of the DNA or nucleic acid during the PCR. In general, however, detection methods for nucleic acids without marking the nucleic acid are also known. So-called gravimetric sensors can be equipped with capture oligonucleotides, thus affording the possibility of detecting the binding event between the nucleic acid to be detected and the catcher by means of the associated mass increase on the sensor. The use of a label is not necessary for this type of detection. Thus, by way of example, the publication "Microcantilever resonance-based DNA detection with nanoparticle probes", M. Su et al., Applied Physics Letters 82 (2003) 3562-3564, discloses a system in which capture molecules are immobilized on a cantilever. Once the nucleic acids have hybridized to the capture molecule, a further, gold-labeled detection nucleic acid is added in order to amplify the mass increase effect. By supplying a silver solution, the silver attaches to the hybridized detection nucleic acid, whereby an amplified mass increase is effected. If the cantilever were sufficiently sensitive, the mass increase as a result of the hybridization of the nucleic acid would suffice for the detection of the hybridization process.

A sensor that manages entirely without a label is disclosed in "First results on label-free detection of DNA and protein molecules using a novel integrated sensor technology based on gravimetric detection principles", R. Gabl, et al., Biosensors and Bioelectronics 19 (2004) 615-620. A so-called thin-film resonator (thin-film bulk acoustic resonator, FABR) is used therein in order to be able to measure the hybridization on the sensor surface directly without a label. The sensor comprises a piezoactive layer that is embedded between two electrodes and has contact with an acoustic mirror on a silicon substrate. Capture molecules, e.g. oligonucleotides, are applied on the top electrode. The sensor generally has a resonant frequency in the range of a few GHz. A hybridization of nucleic acids to the capture molecules shifts the resonant frequency of the sensor proportionally to the mass increase. This makes it possible to draw conclusions firstly about the binding event per se (detection, qualitative), and likewise about the quantity of detected DNA (quantitative). The application of FBAR thin film resonators is not restricted to the detection of nucleic acids. Moreover, proteins and other molecules which can bind to specific capture molecules on the sensor surface can also be detected. The shift in the resonant frequency is directly proportional to the mass occupation density of the bound molecules. In comparison with traditional quartz resonators, FBARs afford the advantage of the easy-to-miniaturize design and the compatibility with established CMOS processes. The construction of arrays of FBARs and the use of small sample volumes are likewise made possible by the miniaturized design. FBAR sensor technology makes it possible to achieve a sensitivity in the range of a few ng/cm². By way of example, assuming the complete binding of a thiol-biotin-DNA complex having a length of 25 base pairs from a sample volume of 10 µl on the surface of an FBAR having a size of 200 x 200 µm² a concentration limit of 1 x 10⁻¹¹ mol/l can be detected.

It is an object of the present invention to provide a method for amplification of nucleic acids by means of which, during the PCR, statements about the progression thereof can be made without having to use labeled nucleic acids or primers in the process.
This object is achieved by means of methods in accordance with claims 1 and 11, respectively. The provision of the gravimetric sensor with primers immobilized thereon makes it possible to achieve and to detect a mass increase at the individual sensor positions, during the elongation step of the amplification, and thus to make statements about the amplification. Since the detection principle of the gravimetric sensor is only based on the mass increase on the chip during the hybridization and extension of the primer, it is not necessary to use labeled primers or nucleotides such as is the case in known methods.

The same principle can be applied to a PCR for two complementary nucleic acid strands. In this case, two primers of a primer pair are provided, a first one of which is immobilized on the gravimetric sensor, while a second primer of the primer pair is in solution. After the first cycle and the denaturation of the first duplicated nucleic acids, second primers of the primer pair will be able to hybridize at the extended first primer since they have complementary sequences. It is likewise possible for the extended second primer to hybridize at the first primer on the sensor. These hybridization operations can be detected by means of the gravimetric sensor and correspond to those of a customary PCR.

In one advantageous embodiment of the method according to the invention, an (optionally double-stranded) control nucleic acid of known concentration is provided. A gravimetric control sensor is likewise provided, first control primers of a control primer pair that are at least partly complementary to the two single strands of the control nucleic acid being immobilized on the surface of said control sensor. At the same time as the hybridization of the nucleic acid with the first primer, the control nucleic acid is also hybridized with the first control primer. The first primer and first control primer are extended, the mass increase during the hybridization and the extension of the primer and of the control primer being measured in each case by means of the gravimetric sensor. In a further method step, the concentration of the nucleic acid is determined from the measured mass increases. This is effected according to known methods that are also employed in label-based real-time PCR methods. Examples thereof are the so-called C_{P} or C_{T} method. Ultimately, the concentration of the nucleic acid can be determined in this way without having to use labeled primers.

In a further advantageous embodiment of the method, the gravimetric sensor is a thin-film resonator (film bulk acoustic resonator, FBAR). The mass increase as a result of the hybridization and the extension of the nucleic acid can be measured by means of such sensors. The sensors have a number of advantages for example over micromechanical gravimetric sensors (e.g. cantilevers). First of all, they can be produced by means of established thin-film methods, where the read-out electronics can be realized completely using thin-film technology in the chip. Therefore, the FBARs can be produced expediently and simply, and they can be greatly miniaturized and parallelized. Moreover, FBARs have a high mass sensitivity, whereby additional weights, as known in cantilever-based methods, can be avoided.

The real-time PCR can thus be parallelized to a great extent. This is not possible in the optical methods since only a limited number of dyes are available and interactions or crosstalk during the detection are unavoidable owing to the superposition of the emission wavelengths. On account of the high parallelizability (array capability), on a chip it is possible both to quantify a multiplicity of target DNAs and to realize different positive and negative controls and also individual array positions with regard to correction measurement of disturbing influences (temperature, viscosity etc.).

The invention makes the real-time/online PCR multiplexable without restriction. The degree of multiplexability of a real time PCR is therefore dependent only on the multiplexability of the PCR and not on the real-time detection system or method - as has been the case hitherto.

Other advantages and configurations of the invention emerge from the exemplary embodiments described below in connection with the accompanying drawings:
In the figures:
   Figures 1 to 7 show a schematic sequence of an amplification method,
   Figure 8 shows a schematic illustration of a gravimetric sensor.

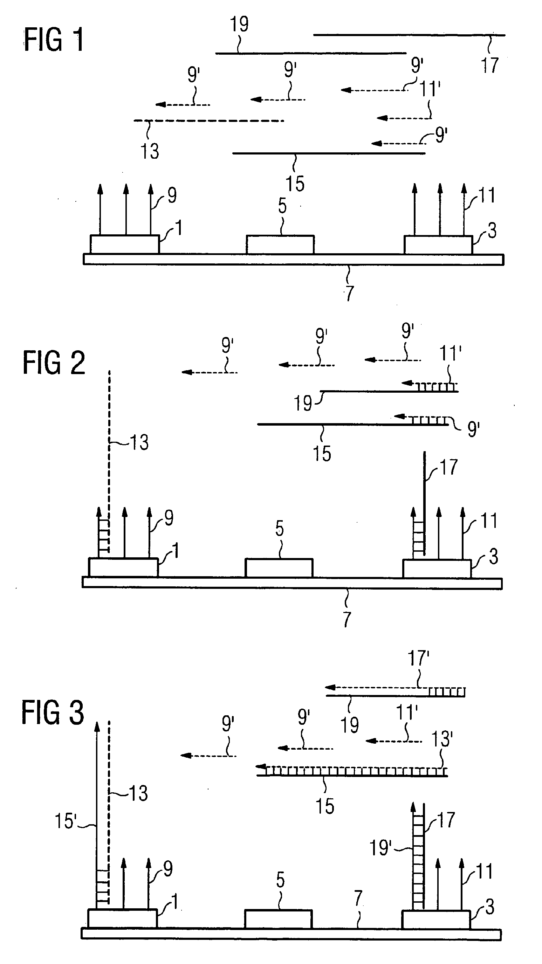

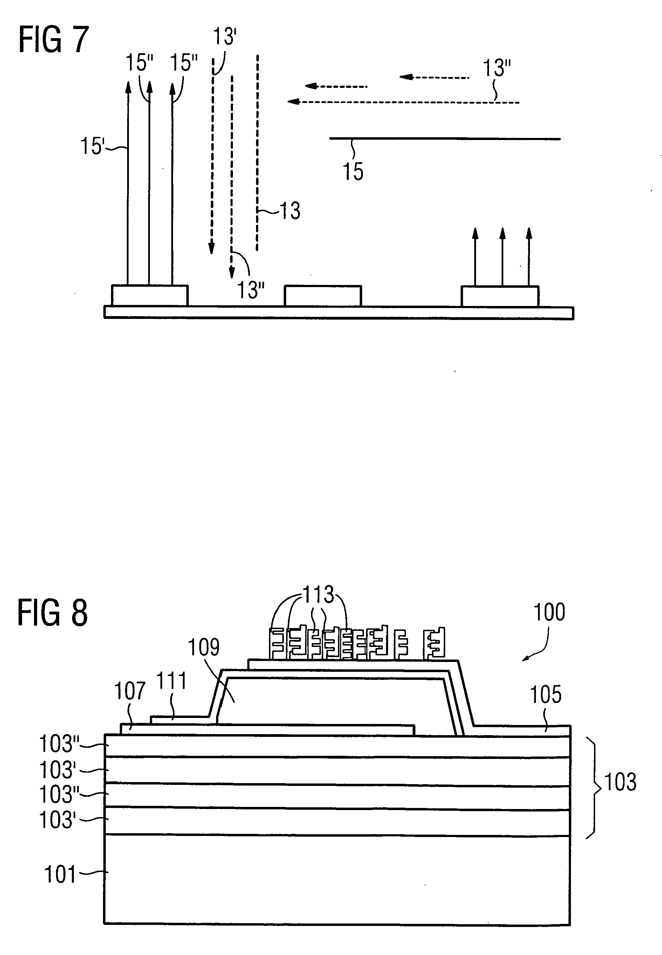

Figures 1 to 7 schematically illustrate two cycles of a preferred embodiment of the method according to the invention. Figure 1 shows sensors 1, 3 and 5, which are applied on a common substrate 7. The sensors are embodied as FBARs, for example. The construction of an FBAR is illustrated schematically in figure 8. The sensors 1, 3 and 5 are thus capable of detecting extremely fine mass increases on their surface. Primers 9, here illustrated schematically as arrows, are immobilized on the surface of the sensor 1. The immobilization can be embodied by means of a thiol-gold binding, for example, which is generally known alongside a multiplicity of other possibilities. The sensor 3 is coated with control primers 11, which are immobilized on the sensor surface in the same way as the primers 9. The sensors 1, 5 and 9 are situated in a common reaction space and are surrounded by a common PCR reaction solution (e.g. 20mM Tris/HCl pH 8.55, 16mM (NH4)2SO4, 0.1-0.5mM dNTPs, 0.5-5mM MgCl2, 0.5-2.5 units Taq polymerase). In solution is a target nucleic acid (DNA) which, by way of example, has been separated (denatured) into its complementary single strands, the nucleic acid 13 and its complementary nucleic acid 15, by heating to a suitable temperature (e.g. 94°C) for a suitable period of time (e.g. 5 min). The separation of the target nucleic acid into its single strands ideally takes place in the reaction space above the sensor chip. The target nucleic acid originates for example from a patient's blood sample that has been isolated by methods performed beforehand. This situation may involve DNA that has been isolated from cells present in the patient's blood (e.g. pathogens), and may likewise involve cDNA that has been obtained from an RNA by means of reverse transcriptase. This is employed for example when determining the viral load of patients infected with HIV.

Ultimately, by means of the described method according to the exemplary embodiment present here, the concentration of the target nucleic acid is intended to be established by means of the concentration of the nucleic acid 13 and 15, respectively. Both concentrations are identical since the nucleic acids 13 and 15 originate from a formerly linked double strand.

The antisense primer 9' associated with the immobilized sense primer 9 is in solution. In addition, the antisense control primer 11' associated with the sense control primer 11 is also present. Two complementary control nucleic acids 17 and 19 are likewise in the solution, which control nucleic acids are present in known concentration and have been produced by denaturation from a double-stranded quantification standard. The control primers 11 and 11' are formed in such a way that they are complementary to the control nucleic acids 17 and 19 and enable, by means of their extension, an amplification of the control nucleic acids.

No oligonucleotides or primers are immobilized on the sensor 2. If a nonspecific mass occupation of the sensors 1, 5 and 9 occurs during the experiment, then this can be detected by means of the sensor 5 in order subsequently to eliminate this interference signal computationally.

Figure 2 illustrates the same arrangement as in figure 1. However, the temperature has been reduced (e.g. to 45 - 60°C), thereby enabling the nucleic acid 13 to combine with the primer 9. The nucleic acid 15 has likewise combined with the primer 9'. The same applies to the control nucleic acids 17 and 19 and the control primer 11 and 11'. The competing hybridization of the nucleic acids 13 and 15, and of the control nucleic acids 17 and 19 among one another, is virtually negligible since the primers 9 and 9', and 11 and 11' are present in excess and annealing between the short-chain primers 9 and 9', and 11 and 11' and the DNA to be amplified is thermodynamically preferred relative to the hybridization of the target DNA strands.

Figure 3 shows the arrangement in the state for example after use of a DNA polymerase at a temperature of approximately 70°C. By means of the DNA polymerase, as in PCR performed as standard, the primers 9 and 11, and also 9' and 11' have been extended to form PCR products 13', 15', 17' and 19', wherein the sequences of the nucleic acids 13 and 15 have been complementarily copied. The same applies to the control nucleic acids 17 and 19.

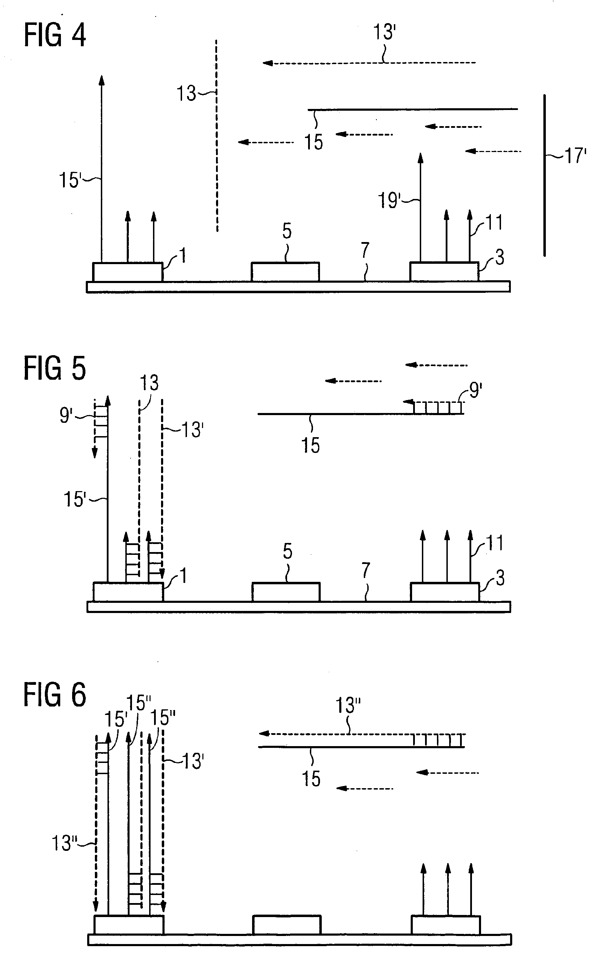

In figure 4, the temperature has been increased again in such a way that a denaturation (melting) of the double-stranded DNA into single strands occurs. Consequently, the originally attached nucleic acid 13 is detached from the PCR product 15', which remains bound on the sensor surface. The double stranded nucleic acid 15 and PCR product 13' are also separated. In comparison with the situation in figure 1, now a copy of the nucleic acid 15, as sensor-bound PCR product 15', and a copy of the nucleic acid 13, as free PCR product 13, are present in each case. The same applies analogously to the control nucleic acids 17 and 19 and their PCR products 17' and 19'.

In figure 5, the temperature has been decreased again in such a way that attachment of nucleic acids can occur. In this case, one of the primers 9' attaches to the nucleic acid 15. Likewise, both the nucleic acid 13 and the PCR product 13' from the first PCR cycle attach to the primers 9 on the sensor 1. A primer 9' that has not yet been extended can now attach to the PCR product 15'. The control primers analogously hybridize with the control nucleic acids and the PCR products thereof, which is not illustrated here for reasons of clarity.

Figure 6 then shows the arrangement after use of the DNA polymerase at temperatures of approximately 70°C. The hybridized primers have been extended analogously to figure 3. This gives rise to PCR products 13" and 15", and also PCR products (not illustrated) from the control primers 11 and 11'.

Figure 7 shows the arrangement after renewed denaturation (end of the second cycle). The number of nucleic acids present in each case has doubled again, such that now four copies of the nucleic acid 13 are present (original nucleic acid 13 and PCR products 13' and 13"). The same applies to the nucleic acid 15 and the control nucleic acids 17 and 19.

The reaction is illustrated by way of example for only one initial copy of the nucleic acids to be examined (double strand of complementary single strands 13 and 15), and of the control nucleic acid sequence (double strand of complementary single strands 17 and 19). Although the methods described can also be carried out with a small number of nucleic acids, generally a multiplicity of specimens of the nucleic acids and different species of nucleic acids in unknown concentration will be present. Likewise, a multiplicity of specimens of the control nucleic acids will be present, although in known concentration. The concentration of the primers 9, 9', 11 and 11' will likewise be higher than is illustrated in the figures. In the case of the reaction illustrated in a simplified manner in figures 1 to 7, the efficiency of the reaction is 100%, which means a doubling of the nucleic acids 13 and 15, and the control nucleic acids 17 and 19 with each PCR cycle. The real efficiency will be lower as a result of incomplete hybridization prior to the respective extension and as a result of competing hybridization between PCR products which have already been formed and in which extension can then no longer take place.

In order, then, to determine the actual efficiency and thereby deduce the concentration of the nucleic acid originally present in the sample (double strand of complementary single strands 13 and 15), use is made of the control nucleic acid (double strand of complementary single strands 17 and 19) which is added in known concentration and amplified in the same PCR. By means of the gravimetric sensor 3, it is possible to measure the mass increase during each PCR cycle and thus to determine the efficiency of the PCR. By measuring the mass increase on the sensor 1, with inclusion of the efficiency determined, it is possible to calculate the original concentration of the nucleic acids 13 and 15.

The method steps described with reference to figures 1 to 7 are comparable to the method steps of a known real-time PCR. They differ from a known real-time PCR in that the primers used here do not have to have a label. A further difference is the immobilization of the primers 9 and 11 on the sensors 1 and 3, whereby it is possible to measure in each case the mass increase as a result of hybridization and extension of the primers 9 and 11.

Figure 8 schematically illustrates the construction of a thin film resonator 100 using FBAR technology (film bulk acoustic resonator). An acoustic mirror 103 is applied on a silicon substrate 101, said acoustic mirror comprising a plurality of thin films 103' and 103'' of layers having alternately low and high acoustic impedances. Said mirror 103 serves for the acoustic decoupling of substrate and resonator. Between two electrodes 105 and 107 a piezoactive layer 109 is applied in a manner connected to the acoustic mirror 103. A biocompatible protective layer 111 is situated on the piezoelectric layer 109. As a result of an alternating electric field being applied to the electrodes 105 and 107, the resonator is excited to oscillation via the piezoelectric layer. The resonant frequency is determined by the layer thicknesses of the electrodes 105 and 107 and of the piezoelectric layer 109 and also the acoustic impedances thereof. Typical resonant frequencies of the FBAR are within the range of 500 MHz to 10 GHz. In order to be able to operate the FBAR in liquids with sufficiently high quality factors, the crystalline orientation of the piezoelectric layer 109 must be constituted such that shear modes can be excited in the configuration described since said shear modes couple to liquids only poorly. The electrode 107 is treated in such a way that capture molecules 113 (primers) can be immobilized there. Coupling of this additional mass coating results in the detuning of the resonant frequency, which can be correspondingly detected electrically. In J. Weber, W.M. Albers, J. Tuppurainen, M. Link, R. Gabl, W. Wersing, M. Schreiter, "Shear mode FBARs as highly sensitive liquid biosensors", Sensors and Actuators A 128, p. 84 - 88, (2006), it was possible to demonstrate mass resolutions of approximately 2 ng/cm² for an 800 MHz resonator.

## Claims

1. A method for the amplification of at least one nucleic acid, comprising the following method steps:
a) provision of a gravimetric sensor (1, 3, 5), first primers (9, 9') of a primer pair that are immobilized on the surface of the sensor (1, 3, 5) and at least partly complementary to the nucleic acid (13, 15),
b) hybridization of the nucleic acid (13, 15) with the first primer (9, 9'),
c) extension of the first primer (9, 9') and
d) measurement of the mass increase during or after the extending of the first primer (9, 9').

2. The method as claimed in claim 1, in which steps b) to d) are performed at least twice.

3. The method as claimed in claim 1 or 2, further comprising the following method steps:
a) provision of a second primer (9, 9') of the primer pair,
b) separation of the nucleic acid (13, 15) and the extended first primer,
c) hybridization of the extended first primer (9, 9') with the second primer (9, 9') and
d) extension of the second primer (9, 9').

4. The method as claimed in claim 3, in which the second primer (9, 9') is in solution.

5. The method as claimed in claim 1 or 2, further comprising the following method steps:
a) provision of a second primer (9, 9') of the primer pair,
b) hybridization of the second primer (9, 9') with the extended first primer (9, 9'),
c) extension of the second primer (9, 9') and
d) measurement of the mass increase during the extending of the second primer.

6. The method as claimed in claim 5, in which method steps b) and c) are performed simultaneously with method steps b) and c) as claimed in claim 1.

7. The method as claimed in one of the preceding claims, in which hybridization is detected by read-out of the sensor.

8. The method as claimed in one of the preceding claims, further comprising the following method steps:
a) provision of at least one control nucleic acid (17, 19) of known concentration,
b) provision of a gravimetric control sensor, first control primers (11, 11') of a control primer pair that are at least partly complementary to the control nucleic acid (17, 19) being immobilized on the surface of the control sensor,
c) hybridization of the control nucleic acid (17, 19) with the first control primer,
d) extension of the first control primer (11, 11'),
e) measurement of the mass increase during the extension of the first control primer (11, 11') and
f) determination of the concentration of the nucleic acid (13, 15) from the measured mass increases.

9. The method as claimed in one of the preceding claims, in which the sensor (1, 3, 5) is a thin-film resonator (film bulk acoustic resonator, FBAR).

10. The method as claimed in one of the preceding claims, in which a further gravimetric sensor (1, 3, 5) is provided, on the surface of which no primers are immobilized and which is read during the method for control purposes.

11. The method as claimed in one of the preceding claims, in which a further gravimetric sensor (1, 3, 5) is provided, on the surface of which non-extendable primers are immobilized and which is read during the method for control purposes.

12. The method as claimed in one of the preceding claims, in which a further nucleic acid sequence complementary to the nucleic acid sequence is amplified by addition of further primers complementary thereto.

13. A method for the amplification of at least two complementary nucleic acids strands, comprising the following method steps:
a) provision of a gravimetric sensor (1, 3, 5), first primers (9, 9') of a primer pair that are immobilized on the surface of the sensor (1, 3, 5) and at least partly complementary to a first one of the nucleic acid strands (13, 15),
b) provision of a second primer (9, 9') of the primer pair in solution, said second primer being at least partly complementary to the second one of the nucleic acid strands (13, 15),
c) hybridization of the first nucleic acid strand (13, 15) with the first primer (9, 9') and the second nucleic acid strand (13, 15) with the second primer (9, 9'),
d) extension of the first primer (9, 9') and the second primer (9, 9'),
e) measurement of the mass increase during the extending of the first primer (9, 9').

14. The method as claimed in claim 11, in which method steps c) to e) are performed multiply in succession.
